# EUROPÄISCHE PATENTSCHRIFT

(11) **EP 1 732 618 B1**
(45) Veröffentlichungstag und Bekanntmachung des Hinweises auf die Patenterteilung: **03.12.2008**
(21) Anmeldenummer: 05731553.3
(22) Anmeldetag: 23.03.2005
(51) Int. Cl.: A61L 24/00

(54) **VERFAHREN ZUR HERSTELLUNG EINES KNOCHEN-IMPLANTATMATERIALS**
METHOD FOR PRODUCING A BONE IMPLANT MATERIAL
PROCEDE POUR REALISER UN MATERIAU D'IMPLANT OSSEUX

(30) Priorität: 06.04.2004 DE 102004016883
(43) Veröffentlichungstag der Anmeldung: 20.12.2006
(73) Patentinhaber: Aap Biomaterials GmbH & Co. KG, 64807 Dieburg (DE)
(72) Erfinder: KAPS, Hans-Peter, 72076 Tübingen (DE); SATTIG, Christoph, 64807 Dieburg (DE); WÜST, Edgar, 64823 Gross-Umstadt (DE); DINGELDEIN, Elvira, 63303 Dreieich (DE); WAHLIG, Helmut, 64287 Darmstadt (DE)
(74) Vertreter: Blumbach - Zinngrebe
(86) Internationale Anmeldenummer: PCT/EP2005/003075
(87) Internationale Veröffentlichungsnummer: WO 2005/099783

(56) Entgegenhaltungen:
- EP-A- 0 657 178
- WO-A-01/34216
- WO-A-87/05521
- US-A- 4 626 392
- US-A- 4 755 184
- US-A- 4 869 906
- US-B1- 6 746 488

## Beschreibung

Die Erfindung betrifft ein Verfahren zur Herstellung von Knochen-Implantatmaterial mit verbesserter mechanischer Beanspruchbarkeit auf der Basis von Formkörpern aus porösem Implantatmaterial, vorzugsweise porösem Hydroxylapatit-Keramikmaterial sowie nach dem Verfahren hergestellte Implantat-materialien.

Solche Knochen-Implantatmaterialien sowie Verfahren zu ihrer Herstellung sind, z. B. aus der US 4 626 392 A bzw. US 4 869 906 A bekannt.

Die sich bereits seit Jahren abzeichnende zunehmende Bedeutung der Knochenersatzmaterialien hält nicht nur unvermindert an, sondern hat in den letzten Jahren auch noch an Bedeutung gewonnen. Nach verlässlichen Schätzungen wird jährlich in der Orthopädie und Traumatologie in etwa 1,5 Mio. Fällen und im Dentalbereich und in der Kieferchirurgie in ca. 5 Mio. Fällen Knochenersatz verwendet. Ursachen hierfür sind Unfälle, Tumore, Wirbelsäulenerkrankungen oder etwa die Volkskrankheiten Osteoporose und Parodontose.

Für Europa ergeben sich dabei etwa folgende Fallzahlen für eine potentielle Anwendung von Knochenersatz:

| | |
|---|---|
| Orthopädie | ca. 600.000 |
| Kieferchirurgie / Dental | ca. 1,9 Mio. |
| Wirbelsäulenversteifungen und Wirbelkörperersatz | ca. 60.000 |
| Pseudarthrosen | ca. 60.000 |
| Frakturen | ca. 2,5 Mio. |
| Endoprothesenoperationen | ca. 300.000 |
| Arthrose | ca. 7 Mio. |

Die heutigen Methoden der Knochenrekonstruktion weisen gravierende Nachteile auf. Als Goldstandard gilt z. Zt. die Transplantation autologen Knochenmaterials, insbesondere autologer Spongiosa. Ein erheblicher Nachteil dieser Methode besteht in der Notwendigkeit eines zusätzlichen operativen Eingriffs, bei dem körpereigener Knochen vom Patienten entnommen werden muss. Bevorzugte Entnahmestelle ist dabei der Beckenkamm. Dort entsteht aber zwangsläufig ein neuer Knochendefekt, der nicht nur dem Patienten meist erhebliche Beschwerden verursacht, sondern der wünschenswerter Weise mit einem Knochenersatzmaterial aufgefüllt werden sollte. Es versteht sich von selbst, dass durch dieses Verfahren erhebliche Mehrkosten im Krankenhaus und durch Arbeitsausfall entstehen. Darüber hinaus sind bei ca. 20 - 25 % aller so behandelten Patienten mit größeren Komplikationen wie z. B. Infektionen und damit verbundenen Mehrkosten zu rechnen.

Aus diesen Darlegungen ergibt sich eindeutig die Notwendigkeit für die Anwendung "künstlicher", d. h. nicht körpereigener Knochenersatzmaterialien. Die Hauptanwendungsgebiete für Knochenersatzmaterialien liegen also in den Bereichen Orthopädie, Traumatologie einschließlich Schädel-, Gesichts- und Kieferchirurgie. Die wesentlichen Indikationen für Knochenersatzmaterialien betreffen insbesondere die Versorgung großer Knochendefekte, das Auffüllen von Hohlräumen im Skelettsystem, die Verwendung bei Korrekturosteotomien oder bei Versteifungsoperationen, z.B. an der Wirbelsäule und ihre gleichzeitige Verwendung als Trägermaterialien für die lokale Applikation verschiedenster pharmazeutischer und biologischer Wirkstoffe. Eine ausführliche Beschreibung und Würdigung des gegenwärtigen Kenntnisstandes wird in der WO 01/34216 gegeben.

In der klinischen Anwendung haben sich insbesondere Knochenersatzwerkstoffe auf Basis von Hydroxylapatit und Kalziumsulfat bewährt. So wird in der EP 657 178 eine Kalziumphosphatkeramik beschrieben, die aufgrund ihrer positiven chemisch/physikalischen und biologischen Eigenschaften breite klinische Anwendung in den verschiedensten Indikationen für Knochenersatzmaterialien gefunden hat. In dieser Patentschrift wird ein aus tierischem Material in einem besonderen Herstellungsverfahren erzeugtes Implantatkeramikmaterial auf Hydroxylapatitbasis, das unter dem Namen Cerabone^{®} im Handel ist, charakterisiert. Dabei handelt es sich um eine Keramikmatrix mit einem interkonnektierenden Porensystem, das morphologisch der natürlichen Knochenspongiosa entspricht. Das Material besitzt eine Qualität, wie sie von der ASTM für Hydroxylapatitkeramik als Implantationsmaterial gefordert wird.

Wie andere dichte oder poröse Keramikmaterialien, die in den verschiedensten geometrischen Formen (wie Kuben, Zylinder, Kegel, Kugeln, etc.) zur Anwendung gelangen, wird auch das in der Patentschrift EP 0 657 178 beschriebene Material hauptsächlich in Form von Würfeln oder Quader verschiedener Größen klinisch angewandt.

Sowohl in entsprechenden Tierversuchen, als auch im Laufe der langen klinischen Anwendung wurde insbesondere die gute Verträglichkeit, aber auch die biologische Wirksamkeit und der therapeutische Nutzen dieses Keramikimplantatmaterials bewiesen. Im Gegensatz zu bestimmten Knochenersatzmaterialien, die nach ihrer Implantation im Organismus mehr oder weniger schnell abgebaut und im Idealfall auf längere Sicht von körpereigenem neu gebildeten Knochen ersetzt werden, wird eine gesinterte Hydroxylapatitkeramik, wie sie z.B. Gegenstand der EP 0 657 178 ist, nach der Implantation praktisch nicht abgebaut und verbleibt daher nahezu unverändert auf immer im Organismus.

Diese biologische Stabilität, insbesondere wenn sie mit einer bestimmten mechanischen Festigkeit des Implantates selbst verbunden ist, stellt per se keinen Nachteil dar, beschränkt aber die Anwendung eines solchen Materials auf ganz bestimmte medizinische Indikationsgebiete. Im Hinblick auf die knöcherne Integration garantieren poröse Materialien, die, nach passgenauer Implantation, die eine möglichst großflächige innige Verbindung mit dem Wirtsknochen gewährleistet, die besten klinischen Ergebnisse. In solchen Fällen werden innerhalb einiger Monate die einen knöchernen Defekt ausfüllenden Implantate von den Kontaktflächen her von neugebildetem Knochen durchwachsen, der dabei die innere Oberfläche des Porensystems der Implantate wie eine Tapete überzieht. Diese Art der knöchernen Integration führt, wie entsprechende Tierversuche zeigten, im Vergleich zur Ausgangsform zu einer deutlich gesteigerten mechanischen Festigkeit der implantierten Keramik (Hing, K.A., Best, S.M., Tanner, K.E., Bonfield, W., Revell, P.A.: J. Mat. Sci: Mat. in Med. 8, 731-736, 1997: "Biomechanical Assessment of Bone Ingrowth in porous Hydroxyapatite"). Trotzdem ist die der gesinterten Hydroxylapatitkeramik als Werkstoff materialimmanente Sprödigkeit ein unbestreitbarer Nachteil, der ihren medizinischen Anwendungsbereich limitiert und auf ganz bestimmte Indikationen einschränkt. Allerdings haben sich in den entsprechenden Indikationsbereichen diese Implantatmaterialien sehr gut bewährt und dort ihren festen Platz erobert. Insbesondere handelt es sich hierbei um Anwendungsbereiche, bei denen es notwendig ist, meist größere Knochendefekte dauerhaft auszufüllen, eine gewisse Primärstabilität zu erzielen und durch die Durchbauung mit körpereigenem neugebildetem Knochen auf lange Sicht die Ausbildung bindegewebigen Narbengewebes im Defekt zu verhindern. Eine besonders interessante Indikation stellt in dieser Hinsicht z. B. die Pfannendachrekonstruktion beim künstlichen Hüftgelenkersatz dar. Allerdings zeigt gerade diese Indikation sehr klar, dass es dabei notwendig ist, die im Markt befindlichen Keramikblöcke der biologisch/morphologischen Form des aufzufüllenden Defektes mechanisch anzupassen und fest zu verankern, da nur so der für den therapeutischen Erfolg unbedingt notwendige innige Kontakt zu dem Wirtsknochen hergestellt werden kann. Tatsächlich gibt es praktisch überhaupt keinen Anwendungsbereich bei dem nicht von Seiten des Operateurs an der Implantatkeramik Konturkorrekturen zwecks morphologischer Anpassung vorgenommen werden müssen, da nun einmal die biologischen Strukturen der zu behandelnden Knochendefekte fast wie strengen geometrischen Formen folgen. Über die Anpassung der Implantatblöcke an die morphologischen Gegebenheiten des Knochendefektes hinaus ist es jedoch auch noch unbedingt erforderlich, speziell bei größeren Defekten und der Notwendigkeit der Verwendung mehrerer Blöcke, diese untereinander zu fixieren bzw. sie mit dem Knochenlager und mit anderen Osteosynthesematerialien fest zu verbinden, um die medizinisch notwendige Stabilität der Implantate im Defekt zu gewährleisten. Dies ist erforderlich, um Bewegungen der Implantate im Implantatlager sicher auszuschließen, da dies den Einwuchs von körpereigenem neuen Knochengewebe verhindern und die Bildung bindegewebiger Abgrenzungen bedingen würde. Darüber hinaus muss ein fester Kontakt mit dem Wirtsknochenlager hergestellt werden um eine frühzeitige Gefäßversorgung des Implantates vom Implantatlager aus zu erreichen und um das Einwachsen neuen Knochengewebes in das Implantat und damit seine dauerhafte Einheilung im Defektbereich sicher zu stellen. All diese genannten Maßnahmen, die für den therapeutischen Erfolg der Implantation eines Knochenersatzmateriales eine wesentliche Voraussetzung bedeuten, sind aber nur dann optimal möglich, wenn die Keramikblöcke untereinander bzw. im knöchernen Lager des Patienten sicher und fest verbunden, d. h. verschraubt, bzw. z. B. durch Kirchnerdrähte oder durch andere gängige Osteosyntheseverfahren befestigt werden können. Solchen Maßnahmen steht aber die bereits beschriebene Sprödigkeit des Keramikmaterials gegenüber, die es z. B. nicht ermöglicht, einen porösen Hydroxylapatitblock mit einem Schraubengewinde zu versehen und mit einer Schraube fest mit dem Untergrund zu verankern oder mittels eines Kirchnerdrahtes zu fixieren, da das Material dieser mechanischen Beanspruchung nicht gewachsen ist und zerbrechen würde.

Es ist aber gerade diese praktisch immer notwendige mechanische Bearbeitung des Implantatmaterials, in der Regel mit Skalpell, Meisel, Fräse, Bohrer, Säge oder dergleichen, die, wegen der bereits genannten Sprödigkeit der Keramik, meist große praktische Probleme bereitet und nicht selten infolge seiner Zerstörung einen Implantatblock unbrauchbar macht.

Es war daher die Aufgabe dieser Erfindung, Hydroxylapatitkeramikimplantate mit ihren Material-immanenten biologisch sehr positiv zu bewertenden interaktiven Eigenschaften mit dem Wirtsgewebe so zu verändern bzw. zu stabilisieren, dass ihre mechanische Bearbeitbarkeit, insbesondere auch die Möglichkeit der Durchbohrung bzw. der Gewindeschneidung sowie der Anbringung chirurgischer Schrauben oder Drahtbefestigungen, ermöglicht wird. Dem erfinderischen Vorsatz, die mechanische Stabilität einer porösen Hydroxylapatitkeramik entscheidend zu verbessern, lag die Idee zugrunde, das Porensystem des Materials vollständig und durchgängig mit einem hierfür geeigneten Material so zu verfüllen, dass der gewünschte mechanische Effekt erzielt wird. Dabei war aber zu berücksichtigen, dass der für den therapeutischen Erfolg einer knöchernen Integration eines Keramikimplantates so wesentliche Effekt des offenen, konnektierenden Porensystems infolge einer solchen Maßnahme nicht verloren gehen darf. Es bestand also die Aufgabe, einerseits durch eine geeignete Imprägnierung der Keramikmatrix deren mechanische Festigkeit und Bearbeitbarkeit zu erhöhen bzw. zu ermöglichen, andererseits aber den biologischen Effekt ihrer Porosität sicherzustellen.

Erfingdungsgemäß wird dieses Problem dadurch gelöst, dass eine Füllmasse für die Formkörper aus einer pulverförmigen Komponente, die im Wesentlichen aus einer Mischung von Hydroxylapatit- und Kalziumsulfatpulver besteht, wobei das Hydroxylapatitpulver aus synthetisch hergestellten, gefällten hochreinen kristallinen Nano-Partikeln besteht, die eine Kristallgröße von 10 bis 20 nm Breite und 50 bis 60 nm Länge aufweisen, mit Wasser zu einer fließfähigen Konsistenz aufbereitet wird, dass die Formkörper aus porösem Keramikmaterial

in eine mit der Fülllmasse befüllte Mischkammer eingebracht, die Mischkammer dann verschlossen und anschließend der Druck in der Mischkammer unter atmosphärischem Druck abgesenkt wird, worauf die Mischkammer in Rüttelschwingungen versetzt wird,
und dass die in den Poren befindliche Füllmasse dann verfestigt wird. Das erfindungsgemäße Vorgehen soll nachfolgend an einem Beispiel beschrieben werden:
Als Hydroxylapatit wurden Blöcke von Cerabone^{®} benutzt, die entsprechend der Patentschrift EP 0 657 178 hergestellt worden waren. Als Füllmaterial und temporärer Porenverschluss hat sich überraschenderweise ein Materialgemisch aus Hydroxylapatit-Nanokristallen und Kalziumsulfat (nachfolgend als Füllmasse bezeichnet) bewährt, wie es in der WO 01/34216 beschrieben wird. Das dort offenbarte Substanzgemisch, wie es auch zur Herstellung von Formkörpern verwendet wird, hat sich zur Dotierung des Cerabone^{®} sehr gut bewährt. Dieses Material erwies sich deshalb als besonders geeignet, als es zunächst nach der Aufbereitung eine fließfähige plastische Konsistenz aufweist, dann aber in situ aushärtet.

Überraschenderweise hat sich nun gezeigt, dass Cerabone^{®} nach der Imprägnierung mit der Füllmasse ausgezeichnete mechanische Eigenschaften und eine wesentlich gesteigerte Stabilität besitzt. So können solchermaßen präparierte Formkörper nicht nur mit den hierfür geeigneten herkömmlichen chirurgischen Instrumenten in ihren Konturen bearbeitet und so in jede gewünschte Form gebracht werden ohne zu brechen, sie können auch mit Bohrern (von Hand oder mechanisch) oder Drähten und Stiften durchbohrt und mit Gewindeschneidern bearbeitet werden. In die so entstandenen Bohrgänge und Gewinde können Schrauben fest eingedreht werden, die wie entsprechende Auszugsversuche belegen, im Vergleich mit nicht behandelten Cerabone^{®}-Blöcken eine hohe Verankerungsfestigkeit besitzen.

Mit der Füllmasse ist es möglich, erfindungsgemäß das gesamte Hohlraumsystem der Cerabone^{®}-Blöcke homogen zu durchdringen und so zu stabilisieren. In der klinischen Anwendung zeigte die ausgehärtete Füllmasse, implantiert als Formkörper in Knochendefekte, die gleichen positiven biologischen Effekte wie sie für das in der Patentschrift EP 0 664 133 beschriebene pastöse kristalline Hydroxylapatitmaterial beschrieben wurden. Der Wirkungsmechanismus und die biologisch/therapeutische Effizienz, die unerwartet und völlig abweichend von den Erfahrungen mit anderen Hydroxylapatit-Implantaten sind, wird für die Anwendung der ausgehärteten Füllmasse ausführlich in der WO 01/34216 beschrieben. Aus den dort dokumentierten Eigenschaften geht hervor, dass neben anderen wesentlichen medizinisch wichtigen Eigenschaften die Füllmasse die Knochenneubildung im Defektbereich stark anregt und eine rasche Neoangiogenese auslöst. Im Tierversuch zeigen sich schon nach 10 Tagen, also zu einem ungewöhnlich frühen Zeitpunkt, am Implantationsort Vorläufer der knochenbildenden Zellen, die sich im Bereich der Apatit-Nanokristalle absiedeln und dort neuen Knochen bilden. Dabei kommt es zu einem Abbau der Matrix ("Füllmasse"), so dass die Rate der Resorption des Implantates mit der physiologischen Rate der Knochenneubildung sehr gut übereinstimmt.

Dies heißt aber, dass die erfindungsgemäße Verwendung der Füllmasse die Poren des Cerabone^{®} nicht dauerhaft verschließt, sondern im Gegenteil die für die knöcherne Integration des Cerabone^{®} so notwendigen Poren für den Einwuchs von neuem Knochengewebe in einem physiologischen Zeitrahmen freigibt und im Gegensatz zu dem unbeschickten Cerabone^{®} aufgrund ihrer besonderen biologischen Eigenschaften das Wachstum von neuem Knochen im Porensystem anregt, fördert und beschleunigt. Auf diese Weise erweist sich die Kombination von Cerabone^{®} mit der Füllmasse als in bisher unbekannter Weise therapeutisch außerordentlich vorteilhaft.

Ein weiterer therapeutischer Vorteil für die Verwendung der Füllmasse zur Dotierung poröser Keramikblöcke liegt darin, dass es möglich ist (was erfahrungsgemäß bei unbehandelten Blöcken nicht praktikabel ist), über die Beschickung der Füllmasse mit verschiedensten pharmazeutischen Wirkstoffen, wie insbesondere Antibiotika und Wachstumsfaktoren oder Antiphlogistika, die positiven therapeutischen Effekte solcher Wirkstoffe in Verbindung mit der Implantation von Keramikblöcken nutzbar zu machen.

Wie sich allerdings bei der erfindungsgemäßen Dotierung von Cerabone^{®}-Blöcken mit der Füllmasse zeigte, war es mit bekannten und üblichen Verfahren, wie z. B. dem Einpressen oder dem Einsaugen - auch unter Anwendung eines Vakuums - unmöglich, eine gleichmäßige Durchdringung der Keramikblöcke mit der Füllmasse zu erzielen.

Nach umfangreichen Untersuchungen zeigte es sich jedoch überraschenderweise, dass durch Anwendung eines besonderen Rüttelverfahrens unter gleichzeitig verringertem Atmosphärendruck eine vollständige und homogene Auffüllung des Porensystems des Cerabone^{®} mit der Füllmasse gelingt.

Nachfolgend soll an einem Beispiel das neu entwickelte erfindungsgemäße Verfahren näher beschrieben werden:
1. In einem geeigneten Mischbehälter werden 2,86 Teile Ostim^{®} 35 %_{ig} mit 1 Teil Calciumsulfat unter vermindertem Atmosphärendruck ( < 150 Millibar) homogen vermischt.
2. Nach Öffnen des Mischers wird ein Cerabone^{®}-Block in die pastöse Masse eingebracht und der Mischer wieder verschlossen.
3. Unter vermindertem Atmosphärendruck wird der Mischer auf einen hierfür geeigneten Rüttler (z. B. Druckluftrüttler der Fa. Netter NFP25S) verbracht und der Inhalt für 2 Min. gerüttelt.
4. Entnahme des Blockes und Abstreifen der überflüssigen Paste.
5. Trocknen des dotierten Blockes bei 35° C im Trockenschrank für 24 h.

Im Labormaßstab verwendete Geräte:
MixOR^{®} oder EASYMIX^{®}
Druckluftrüttler der Fa. Netter NFP25S

### Besonderheiten:

a) Eine geringe zusätzliche Wasserzugabe ist nötig, da der vorgegebene HA-Gehalt im Ostim^{®} zwischen 36 und 38 % beträgt. Dieser Wasserzuschlag wird vor der Zugabe des Kalziumsulfates unter Vakuum im Rüttler untergemischt. Dabei erniedrigt sich die Viskosität des Ostim^{®} merklich.
b) Der Block muss während des Einrüttelns in Lage gehalten werden, da er sonst aufsteigen kann.
c) Das Porenvolumen im Block errechnet sich aus dem Wassergehalt des Ostim^{®} (Die Menge Wasser, welche zum Abbinden des Kalziumsulfates benötigt wird ist relativ gering).
d) Der Ostim^{®}-Gehalt kann reduziert werden, was eine bedeutend höhere Festigkeit der Matrix zur Folge hat.
e) Der Gesamt-HA-Gehalt errechnet sich aus Ostim^{®} + Cerabone^{®} und würde damit bei z. B. 40 % Ostim^{®}-Anteil immer noch weit über 50 % liegen.
f) Für den Produktionsmaßstab müsste eine Form aus einem Elastomer hergestellt werden, welche viele rechteckige Vertiefungen aufweist.

Diese müssen ca. 4 mm größer als der zu tränkende Block sein. Die Form würde mit einer Glocke mit Vakuumanschluss verschlossen werden. Die Form muss mit einer freischwingenden Grundplatte verbunden sein, an der ein Rüttler befestigt ist. Die benötigte Menge an Paste kann auf alle Kavitäten verteilt werden. Nach Einfüllen der Paste muss vor dem Verschließen der Form ein geeigneter Niederhalter aufgelegt werden.

Die infolge der Beschickung handelsüblicher Hydroxylapatit-Keramikblöcke (wie z.B. Cerabone^{®}) mit der Füllmasse gemäß dem oben aufgeführten Beispiel verbesserten mechanischen Eigenschaften wurden in entsprechenden experimentellen Untersuchungen nachgewiesen.

Auch hierzu seien Beispiele genannt:
In verschiedenen experimentellen Untersuchungsreihen wurden Druck- bzw. Auszugsversuche durchgeführt. Für diese Versuche wurden Cerabone^{®}-Blöcke in der Größe 20 x 20 x 10 mm verwendet. Sie wurden mit der Füllmasse imprägniert bzw. - zum Vergleich - unbehandelt geprüft (Serie A bzw. B).

Für die Druckversuche wurden die Blöcke vollflächig mit einer Geschwindigkeit von 20 mm/Minute bis zum Zusammenbruch belastet und der hierzu benötigte Druck in Newton pro mm² ermittelt.

Die Auszugsversuche wurden ebenfalls an Cerabone^{®}-Blöcken der Größe 20 x 20 x 10 mm durchgeführt, beschickt mit der Füllmasse oder unbehandelt. In die Blöcke wurden je eine Kortikalis-Schraube (Fa. aap Implantate AG, Art.-Nr. SK 3516-40, Ø 3,5 mm, Länge 40 mm, Serie C bzw. D) oder eine Spongiosa-Schraube (Fa. aap Implantate AG, Art.-Nr. Sp 3506-40, Ø 3,5 mm, Länge 40 mm (Serie E bzw. F) eingedreht. Dabei wurde lediglich vorgebohrt; auch das Schneiden eines Gewindes ist möglich, kann aber auch unterbleiben.

Die notwendige Kraft bis zum Ausreißen der Schrauben aus den Keramik-Blöcken wurde bei einer Geschwindigkeit von 20 mm/Min. ermittelt (Mittelwerte in Newton). Alle Proben wurden vor den Messungen für 5 Min. in Wasser eingelegt.

### Ergebnisse:

| | |
|---|---|
| Serie A (unbehandelt) | 812,25 |
| Serie B (beschickt) | 1.327,21 |
| Serie C (unbehandelt) | 202,48 |
| Serie D (beschickt) | 241,31 |
| Serie E (unbehandelt) | 155,76 |
| Serie F (beschickt) | 279,65 |

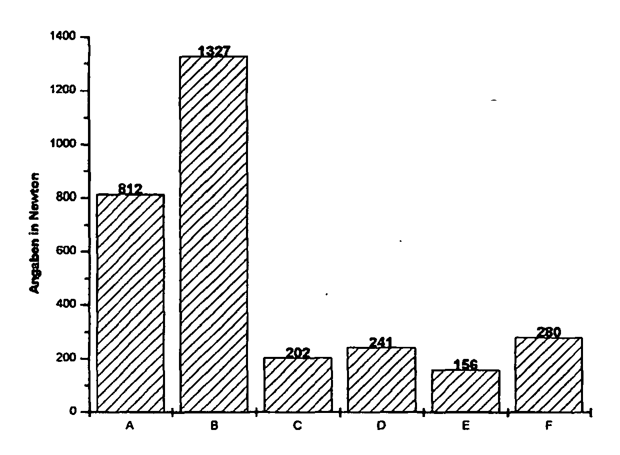

Durch die Beschickung der Keramik-Blöcke mit der Füllmasse konnte die Druckfestigkeit gegenüber unbehandelten Blöcken um 63 % gesteigert werden.

Kortikalis-Schrauben hatten infolge der Beladung eine um 19 % erhöhte Festigkeit gegen Auszug. Bei Spongiosa-Schrauben war die Festigkeit gegen Auszug im Vergleich zu unbehandelten Proben um 79,5 % gesteigert. Die Verankerung der Schrauben in den beladenen Blöcken war spielfrei. Ohne Beladung saßen die Schrauben locker in den Blöcken und konnten bewegt werden.

Durch eine Beschickung von Keramikblöcken mit der Füllmenge war eine wesentliche Steigerung der mechanischen Festigkeit des ansonsten eher spröden Materials möglich, was in der klinischen Anwendung dazu führt, dass eine derartig imprägnierte Keramik mit Osteosynthesematerialien (insbesondere Schrauben) fest und sicher im knöchernen Lager des Patienten fixiert werden kann und außerdem auch infolge seiner deutlich höheren Druckfestigkeit zu einer mechanischen Stabilisierung beiträgt. So könnte z.B. eine derartig festere Keramik in der Wirbelsäulenchirurgie (z.B. Halswirbelsäule) Verwendung finden, während unbehandelte Materialien für diese Anwendung ungeeignet sind.

Darüber hinaus ermöglicht die Beschickung von Keramikblöcken mit der Füllmasse eine mechanische Bearbeitung der Blöcke mit gängigen chirurgischen Instrumenten wie z.B. oszillierender Säge, Bohrer, Meißel, Luer, Feile, Messer etc., die bei unbehandelten Materialien wegen der Sprödigkeit der Keramik nicht möglich ist. So können problemlos die beschickten Blöcke notwendigen Konturkorrekturen mit dem Ziel einer Anpassung an die morphologischen Gegebenheiten des Implantatlagers unterzogen werden.

Neben der beschriebenen Beschickung poröser Keramik-Implantatmaterialien mit einer Mischung aus Kalziumsulfat und Ostim^{®} zwecks Verbesserung ihrer mechanischen und biologischen Eigenschaften, ist die Anwendung dieses besonderen Verfahrens (nämlich einer Dotierung poröser, vorzugsweise spröder oder vergleichsweise weicher, mechanisch wenig stabiler Materialien), auch bei synthetisch hergestellten, porösen Keramiken oder anderen als keramischen Implantatmaterialien, wie z.B. solchen auf Kunststoff-(Polymer-)Basis (PMMA) sinnvoll, da sich dadurch ganz allgemein auch bei solchen Materialien die mechanischen Eigenschaften wesentlich steigern lassen und darüber hinaus die Anwendung der beschriebenen spezifischen Füllmasse zu einer deutlichen Verbesserung der biologisch-medizinischen Wertigkeit der unterschiedlichsten Implantat-materialien, z.B. im Sinne einer besseren und rascheren Durchbauung der Implantate mit neugebildetem Knochen führt. Auch Implantate aus Kalziumsulfat oder Kalziumcarbonat oder anderen organischen Materialien (Lactid/Glycolid, Kollagen) können durch die erfindungsgemäß verwendete Füllmasse in ihren mechanischen Eigenschaften verbessert werden.

## Patentansprüche

1. Verfahren zur Herstellung von Knochen-Implantatmaterial mit verbesserter mechanischer Beanspruchbarkeit auf der Basis von Formkörpern aus porösem Implantatmaterial, vorzugsweise porösem Hydroxylapatit-Keramikmaterial
**dadurch gekennzeichnet,**
**dass** eine Füllmasse für die Formkörper aus einer pulverförmigen Komponente, die im Wesentlichen aus einer Mischung von Hydroxylapatit- und Kalziumsulfatpulver besteht, wobei das Hydroxylapatitpulver aus synthetisch hergestellten, gefällten hochreinen kristallinen Nano-Partikeln besteht, die eine Kristallgröße von 10 bis 20 nm Breite und 50 bis 60 nm Länge aufweisen, mit Wasser zu einer fließfähigen Konsistenz aufbereitet wird,
**dass** die Formkörper aus porösem Keramikmaterial in eine mit der Füllmasse befüllte Mischkammer eingebracht, die Mischkammer dann verschlossen und anschließend der Druck in der Mischkammer unter atmosphärischem Druck abgesenkt wird, worauf die Mischkammer in Rüttelschwingungen versetzt wird, und
**dass** die in den Poren befindliche Füllmasse dann verfestigt wird.

2. Verfahren nach Anspruch 1 **dadurch** gekennzeicnet, dass der Füllmasse vor ihrer Einbringung in das poröse Implantatmaterial wenigstens ein pharmakologisch, biologisch oder chemisch und/oder physikalisch wirksamer Zusatzstoff zugegeben wird.

3. Verfahren nach Anspruch 1 oder 2, **dadurch gekennzeichnet, dass** der Druck in der Mischkammer auf einen Druck < 150 mbar abgesenkt wird.

4. Verfahren nach einem der Ansprüche 1 bis 3, **dadurch gekennzeichnet, dass** die Mischkammer für einen Zeitraum für etwa 2 min. gerüttelt wird.

5. Verfahren nach einem der Ansprüche 1 bis 4, **dadurch gekennzeichnet, dass** die Füllkörper aus der Mischkammer entnommen und überschüssiges Füllmaterial abgestrichen wird.

6. Verfahren nach Anspruch 5, **dadurch gekennzeichnet, dass** die im Porenbereich mit der Füllmasse gefüllten Füllkörper getrocknet werden.

7. Verfahren nach Anspruch 6, **dadurch gekennzeichnet, dass** die Trocknung der in den Poren des Formkörpers eingebrachten Füllmasse während eines Zeitraums von 24 Stunden bei einer Temperatur von etwa 35°C durchgeführt wird.

8. Verfahren nach einem der Ansprüche 1 bis 7, **dadurch gekennzeichnet, dass** die mit den Poren versehenen Füllkörper innerhalb der Mischkammer in dem in die Füllmasse eingetauchten Zustand gegen Aufschwimmen aus der Füllmasse fixiert werden ohne den Zutritt der Füllmasse zu den Füllkörpern zu behindern.

9. Verfahren nach Anspruch 1 oder 2, **dadurch gekennzeichnet, dass** die Aufbereitung der fließfähigen Füllmasse durch Wasserzugabe zu den pulverförmigen Bestandteilen der pulvrigen Komponenten unter Vakuum und Einwirkung von Rüttelschwingungen durchgeführt wird.

10. Implantatmaterial auf der Basis von porösen starren Hydroxylapatit-Keramikfüllkörpem zur Rekonstruktion von Knochendefekten,
**gekennzeichnet durch**
eine in bildsamen Zustand in die Poren der Hydroxylapatit-Keramik-Füllkörper eingebrachte und anschließend verfestigte an sich bekannte Füllmasse, welche aus einer pulverförmigen, aus einer Mischung von Hydroxylapatit-Kalziumsulfatpulver bestehenden Komponente und Wasser aufbereitet ist, wobei das Hydroxylapatitpulver aus synthetisch hergestellten, gefällten hochreinen kristallinen Nano-Partikeln besteht, die eine Kristallgröße von 10 bis 20 nm Breite und 50 bis 60 nm Länge aufweisen.

11. Implantatmaterial nach Anspruch 10, **dadurch gekennzeichnet, dass** die Füllmasse wenigstens einen pharmakologisch, biologisch oder chemisch und/oder physikalisch wirksamen Zustzstoff aufweist.

## Claims

1. Method of producing bone implant material having improved mechanical strength on the basis of moulded bodies consisting of porous implant material, preferably porous hydroxylapatite ceramic material, **characterised in that**
a filling compound for the moulded bodies consisting of a powdery component which consists substantially of a mixture of hydroxylapatite and calcium sulphate powder, wherein the hydroxylapatite powder consists of synthetically produced, precipitated, high-purity, crystalline nano particles which have a crystal size of 10 to 20 nm in width and 50 to 60 nm in length, is prepared with water to produce a flowable consistency,
**in that** the moulded bodies consisting of porous ceramic material are introduced into a mixing chamber which is filled with the filling compound, the mixing chamber is then closed and the pressure in the mixing chamber is then lowered below atmospheric pressure, whereupon the mixing chamber is caused to oscillate, and **in that** the filling compound located in the pores is then solidified.

2. Method as claimed in claim 1, **characterised in that** at least one pharmacologically, biologically or chemically and/or physically effective additive is added to the filling compound prior to introduction thereof into the porous implant material.

3. Method as claimed in claim 1 or 2, **characterised in that** the pressure in the mixing chamber is lowered to a pressure < 150 mbar.

4. Method as claimed in any one of claims 1 to 3, **characterised in that** the mixing chamber is oscillated for a period of about 2 minutes.

5. Method as claimed in any one of claims 1 to 4, **characterised in that** the filling bodies are removed from the mixing chamber and excess filling material is discarded.

6. Method as claimed in claim 5, **characterised in that** the filling bodies which are filled with the filling compound in the pore region are dried.

7. Method as claimed in claim 6, **characterised in that** drying of the filling compound which is introduced in the pores of the moulded body is performed over a period of 24 hours at a temperature of about 35°C.

8. Method as claimed in any one of claims 1 to 7, **characterised in that** the filling bodies which are provided with the pores are fixed inside the mixing chamber when immersed into the filling compound to prevent them from floating up out of the filling compound, without hampering entry of the filling compound into the filling bodies.

9. Method as claimed in claim 1 or 2, **characterised in that** the preparation of the flowable filling compound by the addition of water to the powdery constituents of the powdery components is performed under vacuum and under the effect of oscillations.

10. Implant material on the basis of porous rigid hydroxylapatite ceramic filling bodies for the reconstruction of bone defects,
**characterised by**
a filling mass which is known per se, is introduced in the ductile state into the pores of the hydroxylapatite ceramic filling bodies and is subsequently solidified and which is prepared from a powdery component, consisting of a mixture of hydroxylapatite calcium sulphate powder, and water, wherein the hydroxylapatite powder consists of synthetically produced, precipitated, high-purity, crystalline nano particles which have a crystal size of 10 to 20 nm in width and 50 to 60 nm in length.

11. Implant material as claimed in claim 10, **characterised in that** the filling compound comprises at least one pharmacologically, biologically or chemically and/or physically effective additive.

## Revendications

1. Procédé pour la fabrication de matériau d'implant osseux présentant une possibilité de sollicitation mécanique améliorée à base de corps moulés en matériau d'implant poreux, de préférence du matériau céramique poreux d'hydroxylapatite,
**caractérisé en ce que**,
une masse de remplissage pour les corps moulés à base d'un composant pulvérulent, qui se compose essentiellement d'un mélange de poudre d'hydroxylapatite et de poudre de sulfate de calcium, la poudre d'hydroxylapatite étant à base de nanoparticules cristallines, très pures, précipitées et fabriquées de façon synthétique, qui présentent une grandeur de cristal de 10 à 20 nm de large et de 50 à 60 nm de long, est traitée avec de l'eau pour obtenir une consistance fluide,
**en ce que** les corps moulés à base de matériau céramique poreux sont introduits dans une chambre de mélange remplie avec la masse de remplissage, la chambre de mélange est fermée alors et la pression dans la chambre de mélange est abaissée ensuite sous pression atmosphérique, après quoi la chambre de mélange est exposée à des secousses vibrantes, et
**en ce que** la masse de remplissage se trouvant dans les pores est consolidée ensuite.

2. Procédé selon la revendication 1, **caractérisé en ce que** au moins un produit additif efficace au plan pharmacologique, biologique ou chimique et/ou physique est ajouté à la masse de remplissage avant son introduction dans le matériau d'implant poreux.

3. Procédé selon la revendication 1 ou 2, **caractérisé en ce que** la pression dans la chambre de mélange est abaissée à une pression inférieure à 150 mbars.

4. Procédé selon l'une quelconque des revendications 1 à 3, **caractérisé en ce que** la chambre de mélange est remuée pendant une période d'environ 2 minutes.

5. Procédé selon l'une quelconque des revendications 1 à 4, **caractérisé en ce que** les corps de remplissage sont enlevés de la chambre de mélange et du matériau de remplissage excédentaire est enlevé par raclage.

6. Procédé selon la revendication 5, **caractérisé en ce que** les corps de remplissage remplis dans la zone de pores avec la masse de remplissage sont séchés.

7. Procédé selon la revendication 6, **caractérisé en ce que** le séchage de la masse de remplissage introduite dans les pores du corps moulé est effectué pendant une période de 24 heures à une température d'environ 35°C.

8. Procédé selon l'une quelconque des revendications 1 à 7, **caractérisé en ce que** les corps de remplissage dotés des pores à l'intérieur de la chambre de mélange sont fixés dans l'état plongé dans la masse de remplissage contre une flottaison à partir de la masse de remplissage sans empêcher l'accès de la masse de remplissage aux corps de remplissage.

9. Procédé selon la revendication 1 ou 2, **caractérisé en ce que** le traitement de la masse de remplissage fluide est effectué par l'addition d'eau aux constituants pulvérulents des composants poudreux sous vide et sous l'effet de secousses vibrantes.

10. Matériau d'implant à base de corps de remplissage en céramique d'hydroxylapatite rigide et poreux pour la reconstitution de déficiences d'os,
**caractérisé par**
une masse de remplissage déjà connue, introduite dans l'état souple dans les pores des corps de remplissage en céramique d'hydroxylapatite et durcie ensuite, qui est traitée à partir d'un composant pulvérulent, à base d'un mélange de poudre de sulfate de calcium et d'hydroxylapatite et d'eau, la poudre d'hydroxylapatite étant à base de nanoparticules cristallines, très pures, précipitées et fabriquées de façon synthétique, qui présentent une grandeur de cristal de 10 à 20 nm de large et de 50 à 60 nm de long.

11. Matériau d'implant selon la revendication 10, **caractérisé en ce que** la masse de remplissage présente au moins un agent additif efficace sur le plan pharmacologique, biologique ou chimique et/ou physique.
